# EUROPEAN PATENT SPECIFICATION

(11) **EP 4 326 173 B1**
(45) Date of publication and mention of the grant of the patent: **10.09.2025**
(21) Application number: 22721135.6
(22) Date of filing: 22.04.2022
(51) Int. Cl.: A61B 17/32

(54) **SURGICAL CUTTING ASSEMBLY INCLUDING AN ACTUATOR ASSEMBLY SELECTIVELY OPERABLE IN MODES**
CHIRURGISCHE SCHNEIDANORDNUNG MIT EINER AKTUATORANORDNUNG, DIE SELEKTIV IN MODI BETREIBBAR IST
ENSEMBLE DE COUPE CHIRURGICAL COMPRENANT UN ENSEMBLE ACTIONNEUR POUVANT FONCTIONNER SÉLECTIVEMENT DANS DES MODES

(30) Priority: 23.04.2021 US 202163178571 P
(43) Date of publication of application: 28.02.2024
(73) Proprietor: Stryker European Operations Limited, Carrigtwohill, Co. Cork T45 HX08 (IE)
(72) Inventor: SOLA, Julie, Cork, County Cork T23 N884 (IE); O'SHEA, Conor, Church Hill, Innishannon T12Y K6Y (IE); WALSH, Joshua, Castleredmond, Co., Midleton P25 KD85 (IE); SZYMANSKI, Alexander, Clonmel E91 N402 (IE)
(74) Representative: Schott, Jakob Valentin
(86) International application number: PCT/IB2022/053785
(87) International publication number: WO 2022/224218

(56) References cited:
- WO-A1-2014/133664
- WO-A1-2017/163226
- US-A1- 2011 106 143

## Description

### BACKGROUND

Certain surgical instruments are developed for use with surgical procedures in which access, maneuverability, and visibility are limited. One example is a surgical shaver for procedures of the ear, nose, and throat (ENT), and another example is a surgical bur. The surgical instruments may include a tube assembly in which an inner tube or drive shaft is rotatably disposed within an outer tube, and a cutting window is positioned radially and proximally to a distal tip of the tube assembly. The tissue exposed to the cutting window is therefore based on the orientation of the cutting window and the approach by which the surgeon introduces the surgical instrument into the anatomy. Known devices may undesirably require removing the surgical instrument from the anatomy to reintroduce it in the desired approach. Moreover, the surgical instrument is often designed to be comfortably held in a particular manner, and repositioning in the hand of the surgeon may be unergonomic.

It is further known to include a bend to the tube assembly to provide greater access to certain anatomy. The presence of the bend, however, compounds the aforementioned shortcomings of known devices. Namely, the tissue exposed to the cutting window is further based on the orientation of the bend. For example, the bend may be oriented upwardly relative to the handpiece, and the cutting window may be oriented on a concave side of the tube assembly. For a given approach by which the surgeon introduces the surgical instrument into the anatomy, any number of combinations of the orientation of the cutting window and the orientation of the bend may be preferred, or may become more preferable over the course of the surgical procedure.

Certain known devices provide for orienting the cutting window and/or the bend. One such device is disclosed in WO 2017/163226 A1. It is disclosed that a wheel may be rotated to orient the cutting window. It is further disclosed that the tube assembly may be operably decoupled from the handpiece to selectively orient the bend in one of a limited number of configurations. US 2011/106143 A1 discloses a medical instrument including a handle on the proximal end and a shank rotatably arranged on the handle. A tool is arranged on the distal end of the shank. This tool is rotatable relative to the shank. An actuation element is arranged on the handle. The actuation element may be moved into at least two switch positions, wherein in a first switch position the actuation element is coupled in movement to the shank and in a second switch position the actuation element is coupled in movement to the tool. WO 2014/133664 A1 discloses a blade assembly comprising a tip including two or more tubes, wherein the two or more tubes Include at least: an outer tube and an inner tube; and a mechanical enclosure including: a locking spline having a longitudinal axis; and a slide lock; wherein the slide lock slides along the longitudinal axis of the locking spline and over the locking spline forming a locked state so that rotational movement of the locking spline, around the longitudinal axis of the locking spline, is prevented; and wherein the outer tube is coupled to the locking spline so that rotational movement of the outer tube, around the longitudinal axis of the locking spline, is prevented during the locked state.

Therefore, there is a need in the art for a surgical cutting assembly that provides for selective adjustment of more than one component of the surgical cutting assembly in an intuitive, ergonomic, and efficient manner.

### SUMMARY

The invention is defined in the independent claim. Further developments are set forth in the dependent claims. The surgical cutting assembly of the present disclosure overcomes at least the above shortcomings. The surgical cutting assembly includes an actuator assembly that is operable in at least a first mode and a second mode. A wheel is rotatable in the first mode for adjusting a first component of the surgical cutting assembly, and in the second mode for adjusting a second component of the surgical cutting assembly. The actuator assembly provides for orienting the cutting window in the first mode, and orienting the bend of a tube assembly in the second mode. The wheel is rotatable in a first axial position in the first mode, and in a second axial position in the second mode. The second axial position may be proximal to the first axial position.

The actuator assembly is operably coupled to an intermediate tube in the first mode, and rotating the wheel in the first axial position rotates the intermediate tube. The actuator assembly is operably decoupled from the outer tube in the first mode, and operably decoupled from the intermediate tube in the second mode. The actuator assembly is operably coupled to the outer tube in the second mode, and rotating the wheel in the second axial position rotates the outer tube. The wheel is axially movable relative to the housing between the first mode and the second mode. The actuator assembly includes a biasing member coupled to the wheel. The biasing member biases the actuator assembly to be in the first mode. The actuator assembly may include a first gear, a second gear secured to the outer tube, and a third gear secured to the intermediate tube.

In certain implementations, the actuator assembly includes a first gear, a second gear, and a third gear. The first gear, the second gear, and/or the third gear may be configured to form a Hirth joint. The first gear, the second gear, and/or the third gear, may be a Hirth joint including teeth annularly arranged and oriented proximally or distally to form a face gear with complementary engagement features. Other types of gears may include spur, helical, bevel, worm, screw, and the like. Any suitable joint to be selectively disengaged and reengaged to transmit forces from a rotational input may be provided.

The second gear may be secured to the outer tube at an interface, and the third gear may be secured to the intermediate tube at an interface. The interfaces may be provided by one or more of adhesive, fasteners, friction fit, interference fit, or the like. Rotation of the second gear rotates the outer tube, and therefore the bend of the tube assembly is correspondingly reoriented. Rotation of the third gear rotates the intermediate tube, and with the intermediate tube defining the cutting window, the cutting window of the tube assembly is correspondingly reoriented. The rotation of the wheel with the actuator assembly in the first mode is configured to rotate the third gear, and the rotation of the wheel with the actuator assembly in the second mode is configured to rotate the second gear.

The wheel may include a distal wheel portion and a proximal wheel portion. The distal wheel portion and the proximal wheel portion may be discrete components separately formed and secured to one another to form the wheel. The distal wheel portion and the proximal wheel portion may include joining features configured to facilitate the assembly of the wheel. With the distal wheel portion and the proximal wheel portion secured to one another, the first cavity and the second cavity are sized to accommodate the subcomponents of the actuator assembly, namely the first gear, the second gear, the third gear, the biasing element, and the bearings. The distal wheel portion may include a contour different than a contour of the proximal wheel portion to form a flared surface. The flared surface may be considered more vertically oriented to provide for a larger contact area to pinch and pull the wheel with the thumb and the index finger.

According to a second aspect, an actuator assembly for a surgical instrument is provided. A wheel defines a cavity, and a first gear, a second gear, and a third gear are disposed within the cavity. The second gear is secured to a first subcomponent, and a third gear secured to a second subcomponent. The first component may be an outer tube for any surgical instrument, and the second component may be a shaft, for example a solid drive shaft or another tube. The second component may be the solid shaft, and an end effector may be reoriented. Such examples include a radially disposed electrode for ablation, an eccentrically deployed balloon for kyphoplasty, a radial side port for vertebroplasty, and the like. In such an arrangement, the inner tube may be optional.

The first gear may be selectively engaged with the housing. The second gear may be selectively engaged with the wheel. The third gear may be selectively engaged with the wheel. The first gear includes engagement features that are configured to engage with engagement features of the nose portion of the housing. The first gear may include a first ring with the engagement features being teeth oriented distally and annularly arranged about the first ring. The first gear is sized to be positioned within the first cavity defined by the distal wheel portion. The distal surface of the distal wheel portion defines an opening through which the engagement features of the first gear extend, and the first ring is in interference engagement with an inner surface defining the first cavity. The engagement features of the nose portion may be teeth oriented proximally and annularly arranged in a manner complementary to the teeth of the first gear. With the engagement features of the first gear extending distal to the distal surface of the distal wheel portion, the complementary engagement features are configured to be in selective engagement based on the axial position of the wheel.

The first gear and the second gear may include complementary keying features configured to prevent relative rotation and permit relative translation between the first gear and the second gear. The keying feature of the first gear may be at least one rail, and the keying feature on the second gear may be at least one slot. The first ring of the first gear defines an opening, and the rail is arranged axially within the opening. The neck of the second gear has an outer diameter less than an inner diameter of the opening of the first ring such that the complementary keying features provide for axial translation but not rotation between the first gear and the second gear in each of the first mode and the second mode.

The second gear includes engagement features configured to engage the engagement features of the distal wheel portion. The second gear may include a second ring from which the neck extends distally, and the engagement features of the second gear may be teeth annularly arranged and oriented distally about the second ring. The third gear includes engagement features configured to engage the engagement features of the proximal wheel portion. The third gear may include a third ring, and the engagement features of the second gear may be teeth annularly arranged and oriented proximally about the third ring.

### BRIEF DESCRIPTION OF THE DRAWINGS

Advantages of the inventions will be readily appreciated with the written description being considered in connection with the following drawings.
FIG. 1 is a front perspective view of a surgical instrument including a surgical cutting assembly removably coupled from a capital assembly.
FIG. 2 is a partially exploded rear perspective view of the surgical cutting assembly.
FIG. 3 is a partially exploded front perspective view of the surgical cutting assembly.
FIG. 4 is a sectional elevation view of the surgical cutting assembly including a housing, an actuator assembly, and a tube assembly. The actuator assembly is in a first mode. A cutting window of the tube assembly is oriented on a convex side of the tube assembly.
FIG. 5 is a sectional elevation view of the surgical cutting assembly in which the actuator assembly is in a second mode. The cutting window of the tube assembly is oriented on a concave side of the tube assembly, and the bend of the tube assembly is oriented downwardly relative to the housing.
FIG. 6 is a sectional elevation view of the actuator assembly in the first mode with a portion of the tube assembly shown.
FIG. 7 is a sectional elevation view of the actuator assembly in the second mode with the portion of the tube assembly shown.

### DETAILED DESCRIPTION

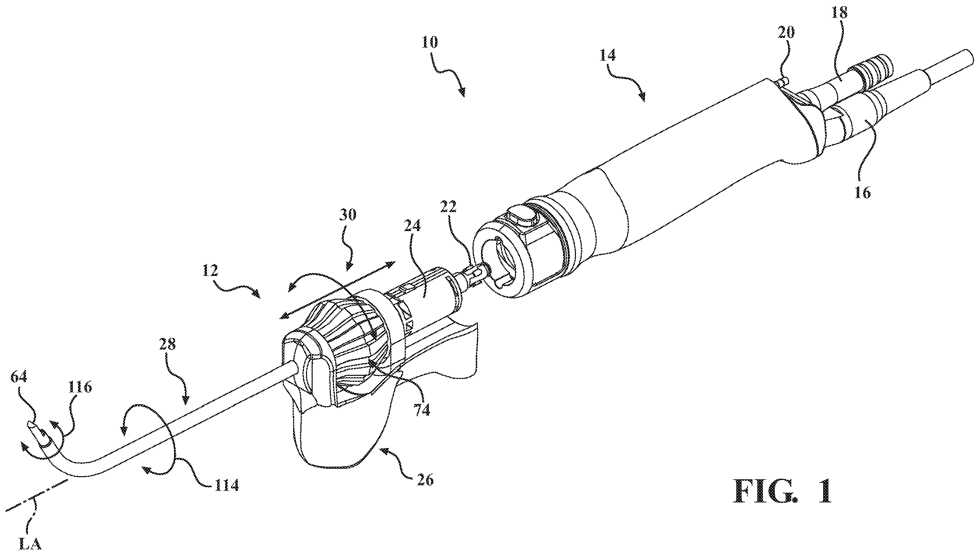

FIG. 1 shows a surgical instrument 10 including a surgical cutting assembly 12 and a capital assembly 14. The capital assembly 14 includes a power line 16 configured to be arranged in electrical communication with a power source, a suction line 18 configured to be arranged in fluid communication with a suction source, and an irrigation line 20 configured to be arranged in fluid communication with a fluid source. An electric motor (not shown) is powered by the power line 16. The capital assembly 14 includes geometries (not shown) configured to operably couple to a drive hub 22 of the cutting assembly 12 with a connecting hub 24 of the cutting assembly 12 supported by and selectively locked with the capital assembly 14. Certain details of the capital assembly 14 are further disclosed in WO 2021/224862 A2, published November 11, 2021, and the aforementioned International Publication WO 2017/163226 A1.

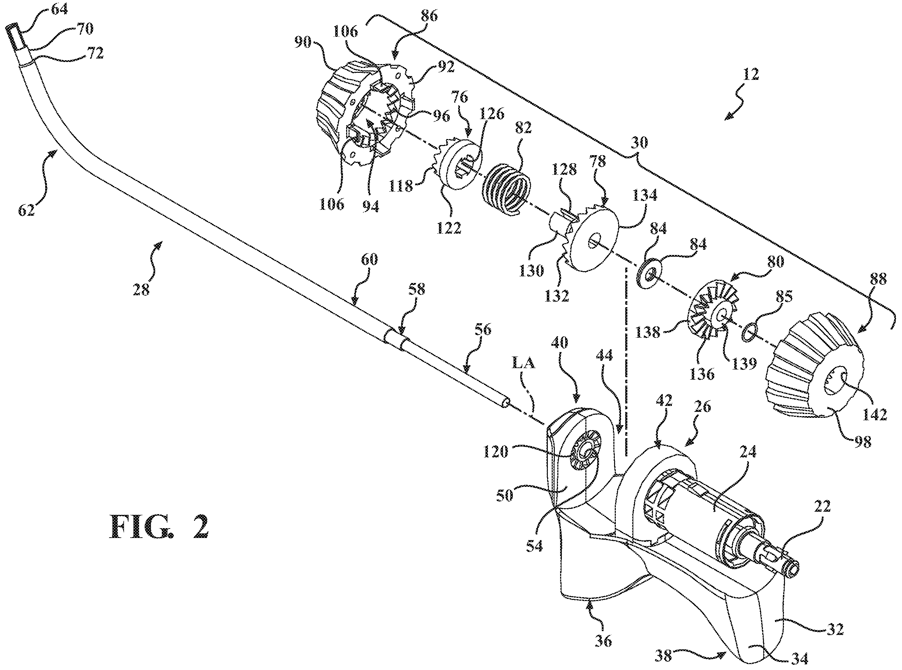

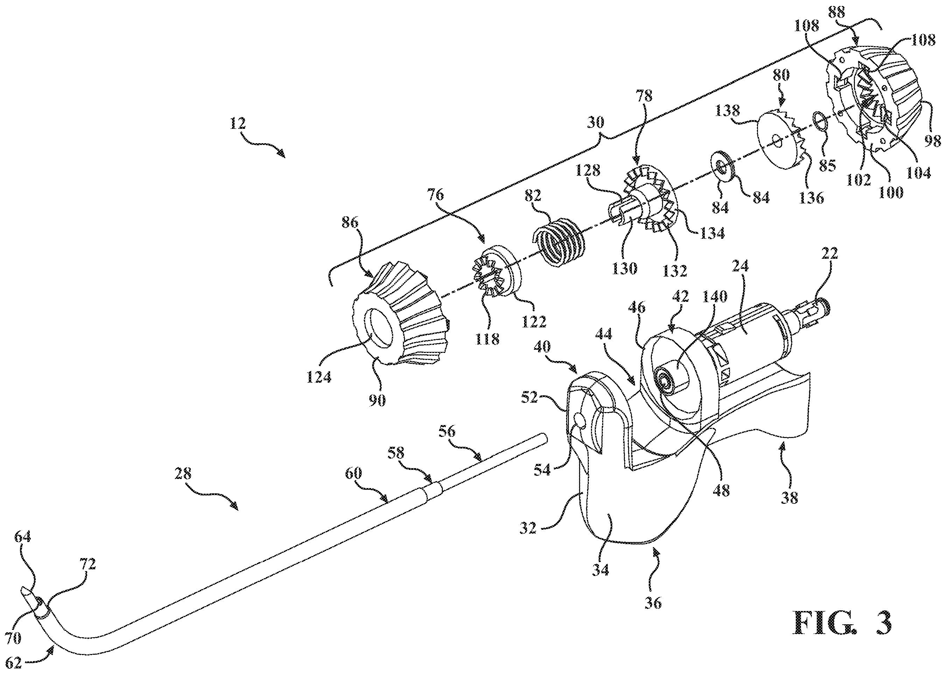

With further reference to FIGS. 2 and 3, the cutting assembly 12 includes a housing 26, a tube assembly 28, and an actuator assembly 30. The housing 26 may include opposing portions or halves 32, 34 coupled to one another to provide a form factor designed to be ergonomic and facilitate one-handed operation of the surgical instrument 10. The opposing portions 32, 34 may also reduce manufacturing complexities and thereby permit cost-effective fabrication of the housing 26 with lower cost materials. Notwithstanding its advantageous functionality to be described, the cutting assembly 12 may be a disposable component and therefore obviate the need for sterilization, reprocessing, sterile handling, and the like.

The housing 26 may include a finger portion 36 and a web portion 38 spaced apart and contoured to ergonomically support a hand of the surgeon. In particular, the finger portion 36 and the web portion 38 may at least partially define a cavity within which a web of the hand of the surgeon may be comfortably supported. Modifications to the housing 26 are contemplated with additional ergonomic arrangements being disclosed in the aforementioned International Publication WO 2017/163226 A1.

The housing 26 includes a nose portion 40, and a collar 42 spaced apart from the nose portion 40 to define a void space 44 sized to receive the actuator assembly 30. The connecting hub 24 is coupled to and extends proximally from the collar 42. The collar 42 comprises a lip 46 to be described, and further defines an opening 48 sized to receive at least an inner tube 56 of the tube assembly 28. The nose portion 40 may extend generally upwardly from the finger portion 36 and include a proximal surface 50 and a distal surface 52 defining an opening 54 therebetween. The opening 54 is sized to receive the tube assembly 28. The proximal surface 50 may be planar so as to be flush with a distal wheel surface 90 of a wheel 74 of the actuator assembly 30. The distal surface 52 may be contoured in any suitable manner to provide an ergonomic support for digits of the hand of the surgeon, particularly if the housing 26 is grasped in a pencil-grip manner.

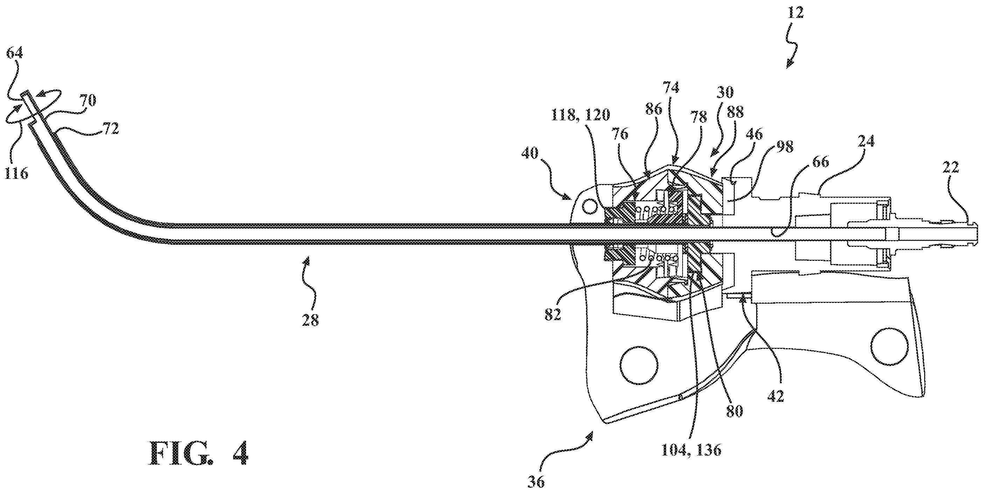

The tube assembly 28 includes the inner tube 56, an intermediate tube 58, and an outer tube 60. The intermediate tube 58 is rotatably and coaxially disposed within the outer tube 60, and the inner tube 56 is rotatably and coaxially disposed within the intermediate tube 58. Owing to respective connections with the actuator assembly 30 to be described, the inner tube 56 may be longer than the intermediate tube 58, and the intermediate tube 58 maybe longer than the outer tube 60. The tube assembly 28 may include a bend 62, and a cutting window 64 positioned distal to the bend 62. More particularly, the intermediate tube 58 may be flexible such that the intermediate tube 58 and the outer tube 60 include complementary bends that form the bend 62. Further, the inner tube 56 may also be flexible so as to accommodate the bend 62, for example, during high-speed rotation. The cutting window 64 may be defined by the intermediate tube 58 with the inner tube 56 including cutting features (not shown) configured to be rotatably positioned within the cutting window 64. The cutting features may be teeth arranged about an inner cutting window of a surgical shaver, or a bur head of a surgical bur. The inner tube 56 defines a suction pathway 66 (see FIGS. 4-7) in fluid communication with the cutting window 64 and configured to be arranged in fluid communication with the suction source coupled to the capital assembly 14. It is further contemplated that an irrigation pathway 68 may be defined between the inner tube 56 and the intermediate tube 58 and configured to be arranged in fluid communication with the fluid source coupled to the capital assembly 14. The irrigation fluid is discharged from the irrigation pathway 68 at a distal end 70 of the intermediate tube 58 adjacent the cutting window 64. A distal end 72 of the outer tube 58 is disposed proximal to the distal end 70 of the intermediate tube 58, and further disposed distal to the bend 62. For convention, the bend 62 may be considered to apportion the tube assembly 28 into a distal portion and a proximal portion with the proximal portion defining a longitudinal axis (LA) of the cutting assembly 12 (see FIG. 1).

With continued reference to FIGS. 2 and 3, the actuator assembly 30 includes the wheel 74, a first gear 76, a second gear 78, and a third gear 80. The first gear 76, the second gear 78, and/or the third gear 80 may be configured to form a Hirth joint. The first gear 76, the second gear 78, and/or the third gear 80, may be a Hirth including teeth annularly arranged and oriented proximally or distally to form a face gear with complementary engagement features. Other types of gears may include spur, helical, bevel, worm, screw, and the like. Any suitable joint to be selectively disengaged and reengaged to transmit forces from a rotational input may be provided. The actuator assembly 30 may optionally include a biasing member 82, bearings 84, and a seal 85. The bearings 84 are configured to limit friction with relative rotation between the second gear 78 and the third gear 80, and the seal 85 may be an O-ring that prevents egress of irrigation fluid being directed through an irrigation pathway between the inner tube 56 and the intermediate tube 58 to instead be discharged at the surgical site.

The wheel 74 is sized to be rotatably positioned within the void space 44 defined by the housing 26. The wheel 74 may include a distal wheel portion 86 and a proximal wheel portion 88. The distal wheel portion 86 includes the distal wheel surface 90, and a first inner wheel surface 92 defining a first cavity 94. The distal wheel portion 86 may further include engagement features 96 disposed within the first cavity 94. The illustrated implementation shows the engagement features 96 as teeth annularly arranged and oriented proximally within the first cavity 94 of the distal wheel portion 90. The proximal wheel portion 88 includes a proximal wheel surface 98, and a second inner wheel surface 100 defining a second cavity 102. The proximal wheel portion 88 may further include engagement features 104 disposed within the second cavity 102. The illustrated implementation shows the engagement features 104 as teeth annularly arranged and oriented distally within the second cavity 102 of the proximal wheel portion 88.

The distal wheel portion 86 and the proximal wheel portion 88 may be discrete components separately formed and secured to one another to form the wheel 74. The distal wheel portion 86 and the proximal wheel portion 88 may include joining features configured to facilitate the assembly of the wheel 74, preferably in a permanent manner in which the distal wheel portion 86 may not be decoupled from the proximal wheel portion 88. The illustrated implementation shows the joining features as barbs 106 (two identified) on the distal wheel portion 86 that are configured to resiliently deflect and engage slots 108 (two identified) defined by the proximal wheel portion 88. The reverse configuration is contemplated, and it is understood that the securing of the distal wheel portion 86 and the proximal wheel portion 88 may be accomplished through other suitable joining means. With the distal wheel portion 86 and the proximal wheel portion 88 secured to one another, the first cavity 94 and the second cavity 102 are sized to accommodate the subcomponents of the actuator assembly 30, namely the first gear 76, the second gear 78, the third gear 80, the biasing member 82, the bearings 84, and the seal 85.

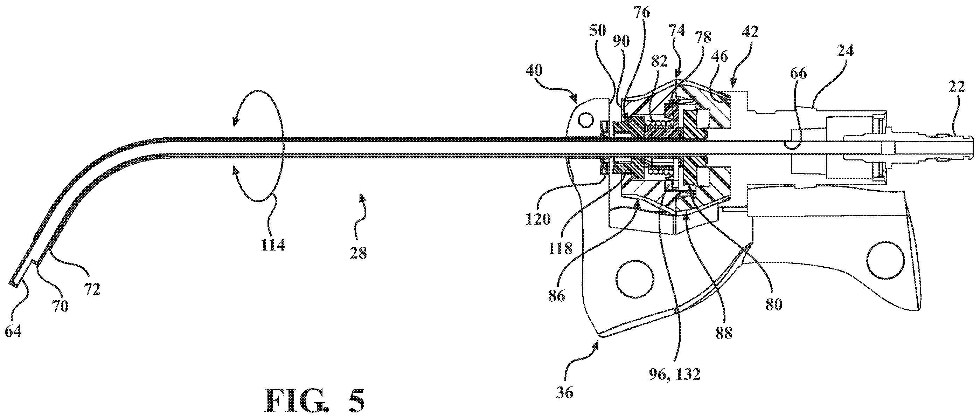

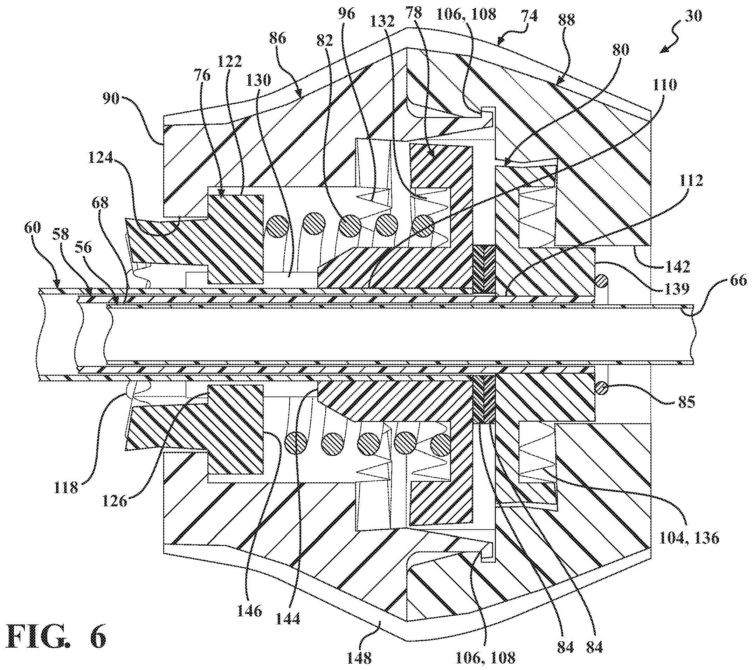

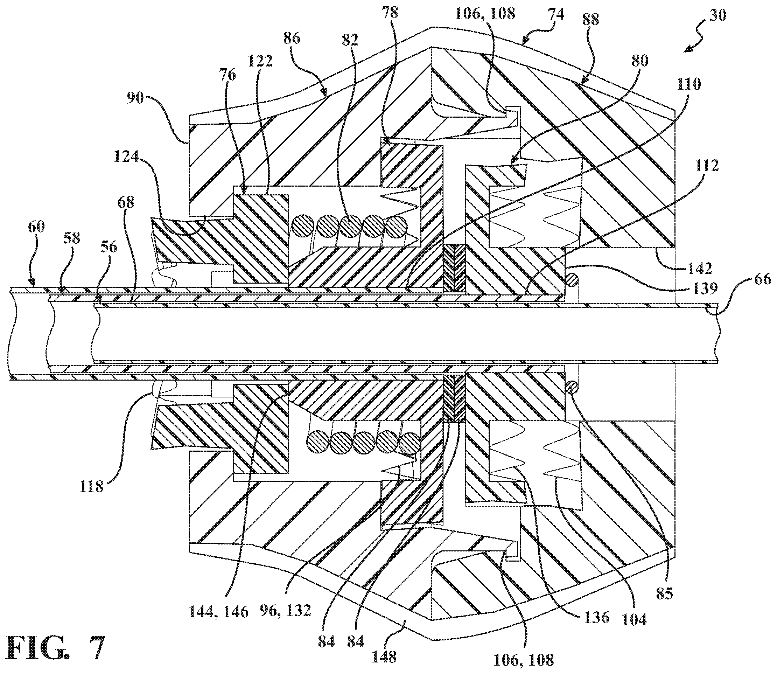

The actuator assembly 30 is selectively operable in at least a first mode and a second mode. FIGS. 4 and 6 show the actuator assembly 30 in the first mode, and FIGS. 5 and 7 show the actuator assembly in the second mode. Operating or actuating the actuator assembly 30 in the first mode is configured to adjust one component of the cutting assembly 12, and operating the actuator assembly 30 in the second mode is configured to adjust another component of the cutting assembly 12. An exemplary arrangement of the actuator assembly 30 provides for orienting the cutting window in the first mode, and orienting the bend in the second mode. More particularly, the wheel 74 may be rotated in the first mode for orienting the cutting window 64, and the wheel 74 may be rotated in the second mode for orienting the bend 62 of the tube assembly 28 about the longitudinal axis. The reverse arrangement is contemplated. The actuator assembly 30 may be selectively moved between the first mode and the second mode by moving the wheel 74 between a first axial position, and a second axial position that is proximal to the first axial position.

The second gear 78 may be secured to the outer tube 60 at an interface 110, and the third gear 80 may be secured to the intermediate tube 58 at an interface 112. The interfaces 110, 112 may be provided by one or more of adhesive, fasteners, friction fit, interference fit, or the like. Rotation of the second gear 78 rotates the outer tube 60, and therefore the bend 62 of the tube assembly 28 is correspondingly reoriented (arrow 114 of FIG. 1). Rotation of the third gear 80 rotates the intermediate tube 58, and with the intermediate tube 58 defining the cutting window 64, the cutting window 64 of the tube assembly 28 is correspondingly reoriented (arrow 116 of FIG. 1). The rotation of the wheel 74 with the actuator assembly 30 in the first mode is configured to rotate the third gear 80, and the rotation of the wheel 74 with the actuator assembly 30 in the second mode is configured to rotate the second gear 78.

Several components of the actuator assembly 30 include engagement features to be described that are configured to facilitate selective engagement and disengagement between the subcomponents in the first mode and the second mode. Referring to FIGS. 2-7, the first gear 76 includes engagement features 118 that are configured to engage with engagement features 120 of the nose portion 40 of the housing 26. The first gear 76 may include a first ring 122 with the engagement features 118 being teeth oriented distally and annularly arranged about the first ring 122. The first gear 76 is sized to be positioned within the first cavity 94 defined by the distal wheel portion 86. More particularly, the distal surface 90 of the distal wheel portion 86 defines an opening 124 through which the engagement features 118 of the first gear 76 extend, and the first ring 122 is in interference engagement with an inner surface defining the first cavity 94 (see FIGS. 6 and 7). The engagement features 120 of the nose portion 40 may be teeth oriented proximally and annularly arranged in a manner complementary to the teeth of the first gear 76. With the engagement features 118 of the first gear 76 extending distal to the distal surface 90 of the distal wheel portion 86, the complementary engagement features 118, 120 are configured to be in selective engagement based on the axial position of the wheel 74. FIG. 4 shows the actuator assembly 30 in the first mode in which the wheel 74 is in the first axial position, and the complementary engagement features 118, 120 being engaged to prevent rotation of the first gear 76 relative to the housing 26. FIG. 5 shows the actuator assembly 30 in the second mode in which the wheel 74 is in the second axial position. A gap is observed between the proximal surface 50 of the nose portion 40 and the distal surface 90 of the distal wheel portion 86, and the complementary engagement features 118, 120 are disengaged from one another. Taken together, the first gear 76 is configured to engage the housing 26 in the first mode, and be disengaged from the housing 26 in the second mode.

The first gear 76 and the second gear 78 may include complementary keying features 126, 128 configured to prevent relative rotation and permit relative translation between the first gear 76 and the second gear 78. The keying feature 126 of the first gear 76 may be at least one rail, and the keying feature 128 on the second gear 78 may be at least one slot. The reverse configuration being contemplated. The illustrated implementation shows two diametrically opposed rails slidably disposed within two diametrically opposed slots defined by a neck 130 of the second gear 78. The first ring 122 of the first gear 76 defines an opening, and the rail is arranged axially within the opening. The neck 130 of the second gear 88 has an outer diameter less than an inner diameter of the opening of the first ring 122 such that the complementary keying features 126, 128 provide for axial translation but not rotation between the first gear 76 and the second gear 78 in each of the first mode and the second mode.

The second gear 78 includes engagement features 132 configured to engage the engagement features 96 of the distal wheel portion 86. The second gear 78 may include a second ring 134 from which the neck 130 extends distally, and the engagement features 132 of the second gear 78 may be teeth annularly arranged and oriented distally about the second ring 134. FIG. 4 shows the actuator assembly 30 in the first mode in which the wheel 74 is in the first axial position and the complementary engagement features 96, 132 axially spaced apart from one another. FIG. 5 shows the actuator assembly 30 in the second mode in which the wheel 74 is in the second axial position and the complementary engagement features 96, 132 engaged with one another. Taken together, the second gear 78 is configured to be disengaged from the distal wheel portion 86 in the first mode, and be engage with the distal wheel portion 86 in the second mode.

The third gear 80 includes engagement features 136 configured to engage the engagement features 104 of the proximal wheel portion 88. The third gear 80 may include a third ring 138, and the engagement features 136 of the third gear 80 may be teeth annularly arranged and oriented proximally about the third ring 138. FIG. 4 shows the actuator assembly 30 in the first mode in which the wheel 74 is in the first axial position, and the complementary engagement features 104, 136 being engaged to provide for rotation of the third gear 80 with rotation of the wheel 74. FIG. 5 shows the actuator assembly 30 in the second mode in which the wheel 74 is in the second axial position and the complementary engagement features 104, 136 are disengaged from one another. Taken together, the third gear 80 is configured to engage the proximal wheel portion 88 in the first mode, and be disengaged from the proximal wheel portion 88 in the second mode.

The third gear 80 includes a proximal surface 139 positioned in an abutting relationship with a boss 140 extending distally from the collar 42 of the housing 26 (see FIG. 3). The proximal wheel portion 88 of the wheel 74 defines an opening 142 sized to be slidably disposed over the boss 140 with the boss 140 abutting the proximal surface 139 of the third gear 80 in the first mode and the second mode. In other words, engagement between the boss 140 of the housing 26 and the third gear 80 may be considered to provide a datum for the axial stack up of the subcomponents of the actuator assembly 30. Moving distally in the sectional view of FIG. 6, the axial stack up includes the bearings 84 disposed between the boss 140 and the third gear 80. The axial stack up further includes the seal 85 disposed between the third gear 80 and the second gear 78 and providing a seal between the boss 140 and the proximal surface 139 of the third gear 80. Consequently, the second gear 78 is axially stationary relative to the third gear 80. The axial stack up further includes the biasing member 82 positioned between the first gear 76 and the second gear 78. The second gear 78 may define an annular recess between the engagement features 132 and the neck 130 with the biasing member 82 being a coil spring having a diameter sized to be positioned within the recess. An opposing end of the coil spring may abut a proximal surface of the first gear 76 with the coil spring configured to bias the first gear 76 distally relative to the second gear 78. More particularly, the biasing member 82 is configured to bias the first ring 122 into interference engagement with the distal wheel portion 86 for functionality previously explained. FIG. 6 shows a distal surface 144 of the neck 130 of the second gear 78 being spaced apart from a proximal surface 146 of the first gear 76, and FIG. 7 shows the distal surface 144 of the neck 130 engaging the proximal surface 146 of the first gear 76 against the spring bias of the biasing member 82.

Actuation of the actuator assembly 30 in the first mode is described with reference to FIGS. 4 and 6. The complementary engagement features 118, 120 are engaged such that the first gear 76 engages the housing 26. The first gear 76 is prevented from rotating relative to the housing 26. The complementary keying features 126, 128 prevent relative rotation between the first gear 76 and the second gear 78, and therefore the second gear 78 is prevented from rotating relative to the housing 26. The engagement features 132 of the second gear 78 are disengaged from the engagement features 96 of the distal wheel portion 86. The engagement features 136 of the third gear 80 are engaged with the engagement features 104 of the proximal wheel portion 88. Consequently, rotation of the wheel 74 results in corresponding rotation of the third gear 80. The third gear 80 is secured to the intermediate tube 58 at the interface 112, and rotation of the third gear 80 results in corresponding rotation of the intermediate tube 58, thereby reorienting the cutting window 64 of the tube assembly 28 (arrow 116). FIG. 4 shows the cutting window 64 rotated 180 degrees relative to FIG. 1 such that the cutting window 64 is on the convex side of the bend 62 of the tube assembly 28.

Should it be indicated to reorient the bend 62 of the tube assembly 28, the actuator assembly 30 is moved from the first mode to the second mode. To actuate the actuator assembly 30 from the first mode to the second mode, an input is provided to the wheel 74 to move the wheel 74 from the first axial position to the second axial position that is proximal to the first axial position. The input may include manipulating the wheel 74 with a thumb and index finger to pinch and pull the wheel 74 proximally against the spring bias from the biasing member 82. To facilitate ergonomic actuation of the actuator assembly 30 from the first mode to the second mode, the distal wheel portion 86 may include a contour different than a contour of the proximal wheel portion to form a flared surface 148. The flared surface 148 may be considered more vertically oriented to provide for a larger contact area to pinch and pull the wheel 74 with the thumb and the index finger.

With reference to FIGS. 5 and 7, the wheel 74 is moved proximally against the spring bias from the biasing member 82. Owing to the aforementioned axial stack up of subcomponents of the actuator assembly 30, the distal wheel portion 86 and the proximal wheel portion 88, including their respective engagement features 96, 104, are moved into and out of engagement with the second gear 78 and the third gear 80, respectively. The distal wheel portion 86, the proximal wheel portion 88, and the first gear 76 move proximally, whereas the second gear 78 and the third gear 80 remain stationary. A proximal aspect of the proximal wheel portion 88 is received by or recessed under the lip 46 of the collar 42. The lip 46 of the collar 42 defines a portion of the void 44 sized to accommodate the wheel 74 when moved to the second axial position with the actuator assembly 30 in the second mode. The lip 46, among other advantages, aesthetically provides for smoother visual transitions between the housing 26 and the wheel 74 by eliminating the appearance of a gap between the housing 26 and the wheel 74 in the first mode, which may be considered the default mode.

With the proximal movement of the wheel 74, the first gear 76 disengages from the housing 26, the second gear 78 engages the distal wheel portion 86, and the third gear 80 is disengaged from the proximal wheel portion 88. More particularly, the engagement features 118 of the first gear 76 are disengaged from the engagement features 120 of the housing 26, the engagement features 132 of the second gear 78 are engaged with the engagement features 96 of the distal wheel portion 86, and the engagement features 136 of the third gear 80 are disengaged from with the engagement features 104 of the proximal wheel portion 88. The disengagement and engagement may be simultaneous. Thereafter, rotation of the wheel 74 results in corresponding rotation of the second gear 78, which is secured to the outer tube 60 at the interface 110. Therefore, the rotation of the second gear 78 results in corresponding rotation of the outer tube 60, thereby rotating the tube assembly 28 about the longitudinal axis (arrow 114) and reorienting the bend 62 of the tube assembly 28 about the longitudinal axis. FIG. 5 shows the bend 62 rotated 180 degrees relative to FIG. 1 with the cutting window 64 is on the concave side of the bend 62 of the tube assembly 28. It may be indicated to maintain the axial position of the wheel 74 against the spring bias from the biasing member 82 to while rotating the wheel 74 in the second mode.

Once satisfied with the orientation of the bend 62 of the tube assembly 28, the input from the surgeon merely need be released to automatically return the actuator assembly 30 from the second mode to the first mode. The biasing member 82, no longer constrained by the input from the surgeon, biases the first gear 76 distally away from the second gear 78, and the interference engagement between the first gear 76 and the distal wheel portion 86 urges the wheel 74 to move from the second axial position to the first axial position. The distal wheel portion 86, the proximal wheel portion 88, and the first gear 76 move distally, whereas the second gear 78 and the third gear 80 again remain stationary. The first gear 76 engages with the housing 26, the distal wheel portion 86 disengages from the second gear 78, and the proximal wheel portion 88 engages the third gear 80. More particularly, the engagement features 118 of the first gear 76 are engaged with the engagement features 120 of the housing 26, the engagement features 96 of the distal wheel portion 86 are disengage from the engagement features 132 of the second gear 78, and the engagement features 104 of the proximal wheel portion 88 are engaged with the engagement features 136 of the third gear 80. The disengagement and engagement may be simultaneous. Rotational input may be immediately provided to the wheel 74 to further orient the cutting window 64 in view of the reorientation of the bend 62. The cutting assembly 12 of the present disclosure provides an intuitive, ergonomic experience that can efficiently be accomplished with a single hand of the surgeon and without distraction from the surgical site.

The foregoing disclosure is not intended to be exhaustive or limit the invention to any particular form. The terminology which has been used is intended to be in the nature of words of description rather than of limitation. Many modifications and variations are possible in light of the above teachings and the invention may be practiced otherwise than as specifically described. One modification may include providing for adjustment of other features of the cutting assembly 12 through the rotatable input the first mode or the second mode. One example includes adjusting a curvature of the tube assembly with actuation of the actuator assembly 30 in the first mode, the second mode, or another mode. It is further understood that the actuator assembly 30 may be designed to be operable in more than two modes. Another modification may include providing for adjustment of components of a surgical instrument through the rotatable input the first mode or the second mode.

In certain implementations, the cutting assembly 12 may include an audible indicator configured to provide audible (and, in some instances, tactile) feedback to the surgeon when the actuator assembly 30 is moved between the first mode and the second mode. The audible indicator may be configured to provide a "clicking" sound when the wheel 74 returns to the first axial position under the spring bias from the biasing member 82, and/or when the wheel 74 has been moved to the second axial position against the spring bias from the biasing member 82. The clicking sound indicates to the surgeon that the actuator assembly 30 has fully returned to the first mode, or has been successfully moved to the second mode. Examples of the audible indicator may include the housing 26 and the wheel 74 having features or components to provide defeatable interference engagement with one another, such as a resiliently deflectable arm, a detent, a radial spring, a cam, a snap, or the like. The engagement and disengagement of the defeatable features or components may provide the clicking sound. In one variant, the biasing member 82 is optional, and the actuator assembly 30 is configured to be snap locked into the first mode and the second mode. For example, the actuator assembly 30 may provide for the defeatable interference engagement between the housing 26 and the wheel 74 in the first axial position and the second axial position. With the actuator assembly 30 in the first mode, for example, the a first defeatable feature may be overcome with sufficient force applied to the wheel 74. The wheel 74 may be freely movable for most of the distance between the first axial position and the second axial position. Upon encountering the a second defeatable feature associated with the second axial position, sufficient force is again applied to the wheel 74. The wheel 74 is snap locked into the second axial position. The clicking sound may be provided with the actuator assembly 30 snap locked into the first mode and the second mode. In still another example, a button or other input may be provided on the housing 26 and/or the wheel 74 to disengage the defeatable features to permit movement of the wheel 74 between the first axial position and the second axial position. Such arrangements obviate the need for the surgeon to maintain the wheel 74 against the spring bias of the biasing member 82, thereby providing a shorter interaction with less fatigue on the fingers of the surgeon.

In certain implementations, a locking mechanism may be provided to selectively prevent the wheel 74 from rotating relative to the housing 26. For example, the housing 26 may include a locking pin or keyed coupling requiring a user input to disengage the locking mechanism from the wheel 74. One exemplary locking mechanism is disclosed in the aforementioned International Publication WO 2017/163226 A1. In certain implementations, visual indicia, such as laser markings, may be provided on the wheel 74, the housing 26, and/or the tube assembly 28 to provide visual information as to the orientation of the bend 62 and/or the orientation of the cutting window 64. For example, the distal wheel portion 86 may be rotatable relative to the proximal wheel portion 88, with the distal wheel portion 86 including a first indicia indicative of the orientation of the bend 62 and the proximal wheel portion 88 including a second indicia indicative of the orientation of the cutting window 64. The first indicia may be an alignment arrow, or a shaped arc to pictorially represent the bend 62 of the tube assembly 28, whereas the second indicia may be an alignment arrow or a shaped oval to pictorially represent the shape of the cutting window 64. In certain implementations, the actuator assembly 30 may be machined in separate parts or molded together. One example includes the distal wheel portion 86 and the proximal wheel portion 88 being integrally formed through a suitable manufacturing processes. It is further contemplated that the housing 26 and the wheel 74 may include different finishes and/or textures to provide a comfortable feel as well as visual indicia of actuatable components of the cutting assembly 12. One exemplary navigation-assisted surgical system suitable with the cutting assembly 12 is disclosed in WO 2016/066287 A1, published May 6, 2016.

## Claims

1. A surgical cutting assembly (12) comprising:
a housing (26);
a tube assembly (28) coupled to said housing (26) and comprising an outer tube (60) extending from said housing (26), an intermediate tube (58) coaxially and rotatably disposed within said outer tube (60), and an inner tube (56) coaxially and rotatably disposed within said intermediate tube (58), wherein said tube assembly (28) further comprises a bend (62), and a cutting window (64) positioned distal to said bend (62); and
an actuator assembly (30) coupled to said housing (26) and said tube assembly (28), said actuator assembly (30) comprising a wheel (74),
**characterized in that** said actuator assembly (30) comprises a biasing member (82) coupled to said wheel (74) and biasing said actuator assembly (30) to a first mode in which said wheel (74) is rotatable in a first axial position to rotate said intermediate tube (58) for orienting said cutting window (64), wherein said wheel (74) is axially movable relative to the housing (26) to a second mode in which said wheel (74) is rotatable in a second axial position to rotate said outer tube (60) for orienting said bend (62).

2. The surgical cutting assembly (12) of claim 1, wherein said actuator assembly (30) further comprises a first gear (76) configured to engage said housing (26) in said first mode, and be disengaged from said housing (26) in said second mode.

3. The surgical cutting assembly (12) of claim 2, wherein said actuator assembly (30) further comprises a second gear (78) secured to said outer tube (60) and configured to disengage from said wheel (74) in said first mode, and be engaged with said wheel (74) in said second mode.

4. The surgical cutting assembly (12) of claim 3, wherein said actuator assembly (30) further comprises a third gear (80) secured to said intermediate tube (58), wherein said third gear (80) is configured to engage said wheel (74) in said first mode, and be disengaged from said wheel (74) in said second mode.

5. The surgical cutting assembly (12) of any one of claims 3 and 4, wherein said first gear (76) and said second gear (78) comprises complementary keying features (126, 128) configured to prevent relative rotation and permit relative translation between said first gear (76) and said second gear (78).

6. The surgical cutting assembly (12) of claim 4, wherein said actuator assembly (30) further comprises a bearing (84) disposed between said second gear (78) and said third gear (80).

7. The surgical cutting assembly (12) of at least claim 3, wherein said biasing member (82) is disposed between said first gear (76) and said second gear (78).

8. The surgical cutting assembly (12) of any one of claims 1-7, wherein said housing (26) comprises a nose portion (40), and a collar (42) spaced apart from said nose portion (40) to define a void (44) sized to receive said wheel (74), wherein said nose portion (40) comprises an engagement feature (120) configured to be engaged by said first gear (76) with said actuator assembly (30) in said first mode.

9. The surgical cutting assembly (12) of claim 8, wherein said collar (42) comprises a lip (46) sized to receive a proximal aspect of said wheel (74) with said actuator assembly (30) in said second mode.

10. The surgical cutting assembly (12) of any one of claims 1-9, wherein said tube assembly (28) defines a suction pathway (66) configured to be arranged in fluid communication with a suction source, and an irrigation pathway (68) configured to be arranged in fluid communication with a fluid source.

11. The surgical cutting assembly (12) of any one of claims 1-10, wherein said housing (26) and said actuator assembly (30) formed a disposable component of said surgical cutting assembly (12).

12. The surgical cutting assembly (12) of any one of claims 1-11, further comprising an audible indicator disposed on at least one of said housing (26) and said wheel (74), said audible indicator configured to provide audible feedback with said actuator assembly (30) assuming one of said first mode and said second mode.

13. The surgical cutting assembly (12) of any one of claims 1-12, further comprising interference features disposed on at least one of said housing (26) and said wheel (74), said interference features configured to be selectively engaged and disengaged to permit movement of said wheel (74) between said first mode and said second mode.

14. A surgical instrument (10) comprising:
said surgical cutting assembly (12) of any one of claims 1-13; and
a capital assembly (14) configured to be removably coupled to said surgical cutting assembly (12), said capital assembly (14) comprising a motor.

15. The surgical instrument of claim 14, wherein said surgical instrument (10) is a shaver or a bur.

## Patentansprüche

1. Chirurgische Schneidanordnung (12), umfassend:
ein Gehäuse (26);
eine Rohranordnung (28), die mit dem Gehäuse (26) verbunden ist und ein Außenrohr (60), das sich vom Gehäuse (26) erstreckt, ein Zwischenrohr (58), das koaxial und drehbar im Außenrohr (60) angeordnet ist, und ein Innenrohr (56), das koaxial und drehbar im Zwischenrohr (58) angeordnet ist, umfasst, wobei die Rohranordnung (28) weiterhin eine Biegung (62) und ein distal zu der Biegung (62) angeordnetes Schneidfenster (64) umfasst; und
eine Aktuatoranordnung (30), die mit dem Gehäuse (26) und der Rohranordnung (28) gekoppelt ist, wobei die Aktuatoranordnung (30) ein Rad (74) umfasst,
**dadurch gekennzeichnet, dass** die Aktuatoranordnung (30) ein Vorspannelement (82) umfasst, das mit dem Rad (74) gekoppelt ist und die Aktuatoranordnung (30) in einen ersten Modus vorspannt, in dem das Rad (74) in eine erste axiale Position drehbar ist, um das Zwischenrohr (58) zum Ausrichten des Schneidfensters (64) zu drehen, wobei das Rad (74) relativ zum Gehäuse (26) axial in einen zweiten Modus bewegbar ist, in dem das Rad (74) in eine zweite axiale Position drehbar ist, um das Außenrohr (60) zum Ausrichten der Biegung (62) zu drehen.

2. Chirurgische Schneidanordnung (12) nach Anspruch 1, wobei die Aktuatoranordnung (30) weiterhin ein erstes Zahnrad (76) umfasst, das so eingerichtet ist, dass es im ersten Modus mit dem Gehäuse (26) in Eingriff steht und im zweiten Modus vom Gehäuse (26) gelöst ist.

3. Chirurgische Schneidanordnung (12) nach Anspruch 2, wobei die Aktuatoranordnung (30) weiterhin ein zweites Zahnrad (78) umfasst, das an dem Außenrohr (60) befestigt ist und so eingerichtet ist, dass es sich im ersten Modus vom Rad (74) löst und im zweiten Modus mit dem Rad (74) in Eingriff steht.

4. Chirurgische Schneidanordnung (12) nach Anspruch 3, wobei die Aktuatoranordnung (30) weiterhin ein drittes Zahnrad (80) umfasst, das an dem Zwischenrohr (58) befestigt ist, wobei das dritte Zahnrad (80) so eingerichtet ist, dass es im ersten Modus mit dem Rad (74) in Eingriff steht und im zweiten Modus vom Rad (74) gelöst ist.

5. Chirurgische Schneidanordnung (12) nach einem der Ansprüche 3 und 4, wobei das erste Zahnrad (76) und das zweite Zahnrad (78) komplementäre Verkeilungsmerkmale (126, 128) umfassen, die so eingerichtet sind, dass sie eine relative Drehung verhindern und eine relative Verschiebung zwischen dem ersten Zahnrad (76) und dem zweiten Zahnrad (78) ermöglichen.

6. Chirurgische Schneidanordnung (12) nach Anspruch 4, wobei die Aktuatoranordnung (30) weiterhin ein Lager (84) umfasst, das zwischen dem zweiten Zahnrad (78) und dem dritten Zahnrad (80) angeordnet ist.

7. Chirurgische Schneidanordnung (12) nach mindestens Anspruch 3, wobei das Vorspannelement (82) zwischen dem ersten Zahnrad (76) und dem zweiten Zahnrad (78) angeordnet ist.

8. Chirurgische Schneidanordnung (12) nach einem der Ansprüche 1 bis 7, wobei das Gehäuse (26) einen Nasenabschnitt (40) und einen Kragen (42) umfasst, der vom Nasenabschnitt (40) beabstandet ist, um einen Hohlraum (44) zu definieren, der so bemessen ist, dass er das Rad (74) aufnehmen kann, wobei der Nasenabschnitt (40) ein Eingriffsmerkmal (120) umfasst, das so eingerichtet ist, dass es vom ersten Zahnrad (76) in Eingriff gebracht wird, wenn sich die Aktuatoranordnung (30) im ersten Modus befindet.

9. Chirurgische Schneidanordnung (12) nach Anspruch 8, wobei der Kragen (42) eine Lippe (46) aufweist, die so bemessen ist, dass sie einen proximalen Teil des Rads (74) aufnimmt, wenn sich die Aktuatoranordnung (30) im zweiten Modus befindet.

10. Chirurgische Schneidanordnung (12) nach einem der Ansprüche 1 bis 9, wobei die Rohranordnung (28) einen Saugpfad (66), der so eingerichtet ist, dass er in Fluidkommunikation mit einer Saugquelle angeordnet ist, und einen Spülpfad (68) definiert, der so eingerichtet ist, dass er in Fluidkommunikation mit einer Flüssigkeitsquelle angeordnet ist.

11. Chirurgische Schneidanordnung (12) nach einem der Ansprüche 1 bis 10, wobei das Gehäuse (26) und die Aktuatoranordnung (30) ein Einwegbauteil der chirurgischen Schneidanordnung (12) bilden.

12. Chirurgische Schneidanordnung (12) nach einem der Ansprüche 1 bis 11, weiterhin umfassend einen akustischen Indikator, der an mindestens einem des Gehäuses (26) und des Rads (74) angeordnet ist, wobei der akustische Indikator so eingerichtet ist, dass er eine akustische Rückmeldung liefert, wenn die Aktuatoranordnung (30) den ersten oder den zweiten Modus einnimmt.

13. Chirurgische Schneidanordnung (12) nach einem der Ansprüche 1 bis 12, weiterhin umfassend Eingriffsmerkmale, die an mindestens einem des Gehäuses (26) und des Rads (74) angeordnet sind, wobei die Eingriffsmerkmale so eingerichtet sind, dass sie selektiv in Eingriff gebracht und gelöst werden können, um eine Bewegung des Rads (74) zwischen dem ersten Modus und dem zweiten Modus zu ermöglichen.

14. Chirurgisches Instrument (10), umfassend:
die chirurgische Schneidanordnung (12) nach einem der Ansprüche 1 bis 13 und eine Hauptanordnung (14), die so eingerichtet ist, dass sie abnehmbar mit der chirurgischen Schneidanordnung (12) verbunden werden kann, wobei die Hauptanordnung (14) einen Motor umfasst.

15. Chirurgisches Instrument nach Anspruch 14, wobei das chirurgische Instrument (10) ein Schaber oder eine Fräse ist.

## Revendications

1. Ensemble de coupe chirurgical (12) comprenant:
un boîtier (26);
un ensemble de tubes (28) accouplé audit boîtier (26) et comprenant un tube extérieur (60) s'étendant à partir dudit boîtier (26), un tube intermédiaire (58) disposé en rotation et de manière coaxiale à l'intérieur dudit tube extérieur (60), et un tube intérieur (56) disposé en rotation et de manière coaxiale à l'intérieur dudit tube intermédiaire (58), dans lequel ledit ensemble de tubes (28) comprend en outre un coude (62), et une fenêtre de coupe (64) positionnée de manière distale par rapport audit coude (62); et
un ensemble actionneur (30) accouplé au boîtier (26) et à l'ensemble de tubes (28), ledit ensemble actionneur (30) comprenant une roue (74),
**caractérisé en ce que** ledit ensemble actionneur (30) comprend un élément de sollicitation (82) accouplé à ladite roue (74) et sollicitant ledit ensemble actionneur (30) dans un premier mode dans lequel ladite roue (74) est rotative dans une première position axiale pour faire tourner ledit tube intermédiaire (58) afin d'orienter ladite fenêtre de coupe (64), dans lequel ladite roue (74) est axialement mobile par rapport au boîtier (26) dans un second mode dans lequel ladite roue (74) est rotative dans une seconde position axiale pour faire tourner ledit tube extérieur (60) afin d'orienter ledit coude (62).

2. Ensemble de coupe chirurgical (12) selon la revendication 1, dans lequel ledit ensemble actionneur (30) comprend en outre un premier engrenage (76) conçu pour s'engrener audit boîtier (26) dans ledit premier mode, et être désolidarisé dudit boîtier (26) dans ledit second mode.

3. Ensemble de coupe chirurgical (12) selon la revendication 2, dans lequel ledit ensemble actionneur (30) comprend en outre un deuxième engrenage (78) fixé audit tube extérieur (60) et conçu pour se désolidariser de ladite roue (74) dans ledit premier mode, et s'engrener à ladite roue (74) dans ledit second mode.

4. Ensemble de coupe chirurgical (12) selon la revendication 3, dans lequel ledit ensemble actionneur (30) comprend en outre un troisième engrenage (80) fixé audit tube intermédiaire (58), dans lequel ledit troisième engrenage (80) est conçu pour s'engrener à ladite roue (74) dans ledit premier mode, et pour se désolidariser de ladite roue (74) dans ledit second mode.

5. Ensemble de coupe chirurgical (12) selon l'une quelconque des revendications 3 et 4, dans lequel ledit premier engrenage (76) et ledit deuxième engrenage (78) comprennent des éléments de clavetage complémentaires (126, 128) conçus pour empêcher la rotation relative et permettre la translation relative entre ledit premier engrenage (76) et ledit deuxième engrenage (78).

6. Ensemble de coupe chirurgical (12) selon la revendication 4, dans lequel ledit ensemble actionneur (30) comprend en outre un palier (84) disposé entre ledit deuxième engrenage (78) et ledit troisième engrenage (80).

7. Ensemble de coupe chirurgical (12) selon au moins la revendication 3, dans lequel ledit organe de sollicitation (82) est disposé entre ledit premier engrenage (76) et ledit deuxième engrenage (78).

8. Ensemble de coupe chirurgical (12) selon l'une quelconque des revendications 1 à 7, dans lequel ledit boîtier (26) comprend une partie nez (40) et un collier (42) espacé de ladite partie nez (40) pour former un vide (44) dont la taille permet de loger ladite roue (74), dans lequel ladite partie nez (40) comprend un élément de mise en prise (120) conçue pour entrer en contact, au moyen dudit premier engrenage (76), avec ledit ensemble actionneur (30) dans ledit premier mode.

9. Ensemble de coupe chirurgical (12) selon la revendication 8, dans lequel ledit collier (42) comprend une lèvre (46) dont la taille permet de loger une face proximale de ladite roue (74) avec ledit ensemble actionneur (30) dans ledit second mode.

10. Ensemble de coupe chirurgical (12) selon l'une quelconque des revendications 1 à 9, dans lequel ledit ensemble de tubes (28) forme une voie d'aspiration (66) conçue pour être en communication fluidique avec une source d'aspiration, et une voie d'irrigation (68) conçue pour être en communication fluidique avec une source de fluide.

11. Ensemble de coupe chirurgical (12) selon l'une quelconque des revendications 1 à 10, dans lequel ledit boîtier (26) et ledit ensemble actionneur (30) forment un composant jetable de l'ensemble de coupe chirurgical (12).

12. Ensemble de coupe chirurgical (12) selon l'une quelconque des revendications 1 à 11, comprenant en outre un indicateur sonore disposé sur au moins l'un parmi ledit boîtier (26) et ladite roue (74), ledit indicateur sonore étant conçu pour fournir un retour d'information sonore lorsque ledit ensemble actionneur (30) adopte l'un dudit premier mode et dudit second mode.

13. Ensemble de coupe chirurgical (12) selon l'une quelconque des revendications 1 à 12, comprenant en outre des éléments d'interférence disposés sur au moins l'un parmi ledit boîtier (26) et ladite roue (74), lesdits éléments d'interférence étant conçus pour être sélectivement en prise et désolidarisés afin de permettre le mouvement de ladite roue (74) entre ledit premier mode et ledit second mode.

14. Instrument chirurgical (10) comprenant:
ledit ensemble de coupe chirurgical (12) selon l'une quelconque des revendications 1 à 13; et
un assemble principal (14) conçu pour être accouplé de manière amovible à l'assemble de coupe chirurgical (12), ledit assemble principal (14) comprenant un moteur.

15. Instrument chirurgical selon la revendication 14, ledit instrument (10) étant un rasoir ou une fraise.
